(19) Europäisches Patentamt
European Patent Office
Office européen des brevets

(11) Veröffentlichungsnummer: **0 269 046 B1**

(12) **EUROPÄISCHE PATENTSCHRIFT**

(45) Veröffentlichungstag der Patentschrift: **22.04.92**

(51) Int. Cl.5: **C07C 67/333**, C07C 69/533

(21) Anmeldenummer: **87117213.6**

(22) Anmeldetag: **23.11.87**

Die Akte enthält technische Angaben, die nach dem Eingang der Anmeldung eingereicht wurden und die nicht in dieser Patentschrift enthalten sind.

# (54) Verfahren zur Isomerisierung von 2-Pentensäureestern zu 3-Pentensäureestern.

(30) Priorität: **27.11.86 DE 3640597**

(43) Veröffentlichungstag der Anmeldung:
**01.06.88 Patentblatt 88/22**

(45) Bekanntmachung des Hinweises auf die Patenterteilung:
**22.04.92 Patentblatt 92/17**

(84) Benannte Vertragsstaaten:
**BE CH DE ES FR GB IT LI NL**

(56) Entgegenhaltungen:
**US-A- 4 327 225**

**PATENT ABSTRACTS OF JAPAN Band 2, Nr. 34, 8. März 1978; & JP-A- 52 133 917**

**JOURNAL OF ORGANIC CHEMISTRY, Bd.47, Nr.14, 2.Juli 1982, American Chemical Society, Seiten 2745-2748; J.HINE et al.: "Double bond stabilizing abilities of formyl, carbo-tert-butoxy and carbomethoxy substituents"**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**W-6900 Heidelberg(DE)**
Erfinder: **Merger, Franz, Dr.**
**Max-Slevogt-Strasse 25**
**W-6710 Frankenthal(DE)**
Erfinder: **Gosch, Hans-Juergen, Dr.**
**Seebacher Strasse 37**
**W-6702 Bad Duerkheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

CHEMICAL ABSTRACTS, Bd.69, Nr.11, 9.Sept.1968, Columbus, Ohio, USA, Seite 4041, Spalte 2, Abstract no. 43226v; D.E. McGREER et al.: "Thermal rearragement of alpha,beta- to beta,gamma-unsaturated esters. Evidence for a 1,5-hydrogen transfer mechanism"

TETRAHEDRON LETTERS, Bd. 25, Nr. 45, 1984, Pergamon Press Ltd., Seiten 5181-5182; Y.IKEDA et al.: " A practical synthesis of (Z)-3-alkenoates"

PATENT ABSTRACTS OF JAPAN, Bd. 6, Nr.7 (C-87) (885) 16.Januar 1982; JP - A - 56 133 243

HELVETICA CHIMICA ACTA, Bd. 64, Nr.94, 1981, Schweizerische Chemische Gesellschaft, Seiten 1023-1025; E.P. KREBS: "94. A note on stereochemical observations of the deprotonation of ethyl 2-alkenoates"

## Beschreibung

Aus Journal of Organic Chemistry, Band 33 (1968), Seiten 1671ff ist bekannt, daß man 2-Pentensäureester photochemisch zu 3-Pentensäureestern isomerisieren kann. Von Nachteil ist bei dieser Arbeitsweise, daß sie einer sehr langen Bestrahlungszeit bedarf um ausreichende Ausbeuten zu erzielen. Außerdem müssen stark verdünnte Lösungen der 2-Pentensäureester verwendet werden, deren Aufarbeitung einen hohen Aufwand bedeutet.

Weiterhin ist bekannt, daß sich 2-Pentensäureester durch metallorganische Basen, wie Lithiumdiisopropylamid (Helv. Chim. Acta, Band 64, (1981), Seiten 1023ff) und Kaliumdisilazid (Tetrahedron Letters, Band 25. (1984), Seiten 5181) in 3-Pentensäureester überführen lassen. Von besonderem Nachteil ist jedoch die sehr hohe Empfindlichkeit der metallorganischen Basen gegenüber Wasser, was eine sehr sorgfältige Arbeitsweise unter Schutzgasatmosphäre erfordert. Weiterhin müssen diese Basen bei sehr tiefen Temperaturen eingesetzt werden, was technisch sehr aufwendig ist.

Entsprechend Can. Journal Chemistry, Band 46 (1968), Seiten 2225ff ist es möglich, 2-Pentensäureester rein thermisch bei Temperaturen über 250 ° C in 3-Pentensäureester umzuwandeln. Hierzu bedarf es jedoch erheblicher Reaktionszeiten, z.B. über 150 Stunden, um einen ausreichenden Umsatz zu erzielen.

In Journal of Organic Chemistry, Band 47 (1982), Seiten 2745 bis 2748 wird auch schon die Isomerisierung von Pentensäureestern mit äquimolaren Mengen 1,8-Diazabicyclo[4,5,0]undec-7-en beschrieben. Es wird jedoch kein Hinweis gegeben, wie die Gewinnung von 3-Pentensäureestern aus 2-Pentensäureestern zu gestalten ist.

Es war deshalb die technische Aufgabe gestellt, ein Verfahren zur Verfügung zu stellen, bei dem man 2-Pentensäureester bei relativ niedrigen Temperaturen in kurzer Zeit möglichst vollständig in 3-Pentensäureester überführt, wobei 4-Pentensäureester, falls sie zugegen sind, möglichst nicht umgewandelt werden.

Diese Aufgabe wird gelöst in einem Verfahren zur Herstellung von 3-Pentensäureestern durch Isomerisierung von 2-Pentensäureestern, wobei man 2-Pentensäureester mit 0,01 bis 0,5 Mol, je Mol Pentensäureester cyclischen, tertiären Aminen mit einem $p_{kA}$-Wert >6 bei einer Temperat von 50 bis 250 ° C behandelt und 3-Pentensäureester fortlaufend abdestilliert.

Das neue Verfahren hat den Vorteil, daß es gelingt, 2-Pentensäureester weitgehend in 3-Pentensäureester umzuwandeln. Weiter hat das neue Verfahren den Vorteil, daß es in relativ kurzer Zeit verläuft und keine aufwendigen Katalysatoren benötigt. Ferner hat das neue Verfahren den Vorteil, daß 4-Pentensäureester praktisch nicht isomerisiert werden.

Das neue Verfahren ist insofern bemerkenswert, als aus der japanischen Patentveröffentlichung 56-55345 bekannt ist, daß 3-Pentensäureester in Gegenwart von 1,8-Diazabicyclo[5,4,0]undecen-7 bei 100 ° C bis zu 60 Mol.% zu 2-Pentensäureester isomerisiert werden. Dies deckt sich mit der allgemein bekannten Tatsache, daß konjugierte Doppelbindungen gegenüber isolierten Doppelbindungen thermodynamisch begünstigt sind. Bemerkenswert ist weiterhin, daß 2-trans-Butensäuremethylester durch Behandeln mit 1,8-Diazabicyclo[5,4,0]undecen-7 unter fortlaufendem Abdestillieren von Butenestern einer Temperatur von 118-120 ° C zu 61 % in 2-cis-Butensäuremethylester und nur zu 9 % in Vinylessigsäuremethylester umgewandelt wird.

Die als Ausgangsverbindungen verwendeten 2-Pentensäureester leiten sich vorzugsweise von Alkanolen mit 1 bis 12 Kohlenstoffatomen, Cycloalkanolen mit 6 bis 8 Kohlenstoffatomen, Aralkanolen mit 7 bis 10 Kohlenstoffatomen oder Phenolen oder Naphtholen ab. Geeignete Ausgangsstoffe sind z.B. die entsprechenden Methyl-, Ethyl-, n-Propyl-, iso-Propyl-, tert.-Butyl-, i-Butyl-, n-Butyl-, sec.-Butyl-, n-Pentyl, n-Hexyl-, Dodecyl-, Cyclopentyl-, Cyclohexyl-, Cyclooctyl-, Benzyl-, Phenylethyl- oder Phenylester. Die als Ausgangsstoffe verwendeten 2-Pentensäureester können in cis- und/oder trans-Form vorliegen. Besonders geeignet sind 2-Pentensäure-alkylester, insbesondere 2-Pentensäure-$C_1$-$C_3$-alkylester.

Die Behandlung führt man bei einer Temperatur von 50 bis 250 ° C durch. Besonders bewährt haben sich Temperaturen von 100 bis 160 ° C.

Die Behandlung führt man in der Regel bei Atmosphärendruck durch. Es ist jedoch auch möglich, die Behandlung unter vermindertem Druck, z.B. bis zu 1 mbar oder schwach erhöhtem Druck, z.B. bis zu 10 bar, durchzuführen.

Die Behandlung erfolgt in Gegenwart von cyclischen, tertiären Aminen mit einem $p_{Ka}$-Wert >6, insbesondere von 6,2 bis 12.

Bevorzugte cyclische, tertiäre Amine sind

a) 5 bis 7-gliedrige, cyclische, tertiäre Amine, die 1 oder

2 Stickstoffatome oder 1 Stickstoffatom und 1 Sauerstoffatom im Ring enthalten können, wobei mindestens ein Stickstoffatom mit einem Alkylrest mit 1 bis 4 Kohlenstoffatomen substituiert ist. Geeignete Verbindungen sind beispielsweise N-Methylpyrrolidin, N-Methylpiperidin, N-Methylhexamethylenimin, N-

Ethylmorpholin, N,N'-Dimethylpiperazin. Besonders bevorzugt sind 5 bis 7-gliedrige, tertiäre, cyclische Amine mit nur einem Stickstoffatom im Ring.

b) Aminopyridine der Formel I

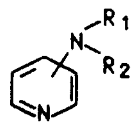

I,

in der $R_1$ und $R^2$ gleich oder verschieden sind und jeweils einen Alkylrest mit 1 bis 4 Kohlenstoffatomen bezeichnen oder gemeinsam mit dem Stickstoffatom, das sie substituierten, einen 5- bis 7-gliedrigen Ring bedeuten können, der zusätzlich ein Sauerstoff- oder Stickstoffatom enthalten kann. Besonders bevorzugt sind Verbindungen der Formel I, die sich vom 4-Aminopyridin ableiten. Geeignete Verbindungen sind beispielsweise N,N-Dimethyl-4-aminopyridin, N,N-Diethyl-4-aminopyridin, 4-Morpholinopyridin oder 4-N-Piperazinopyridin.

c) Imidazole der Formel

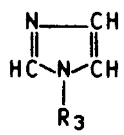

II,

in der $R_3$ einen Alkylrest mit 1 bis 4 Kohlenstoffatomen bezeichnet. Geignete Verbindungen sind beispielsweise N-Methyl-, N-Ethyl-oder N-Propylimidazol.

d) Bicyclische Amidine, insbesondere 1,8-Diazabicyclo[5,4,0]undecen-7, 1,5-Diazabicyclo[4,3,0]non-5-en oder 1,4-Diazabicyclo[2,2,2]octan.

Es versteht sich, daß auch Gemische der vorgenannten cyclischen, tertiären Amine verwendet werden können.

Je Mol 2-Pentensäureester verwendet man 0,01 bis 0,5 Mol, bevorzugt 0,02 bis 0,2 Mol insbesondere 0,05 bis 0,2 Mol cyclisches, tertiäres Amin an.

Die bei der Isomerisierung entstehenden 3-Pentensäureester werden fortlaufend, vorzugsweise in dem Maße, wie sie entstehen, aus dem Reaktionsgemisch abdestilliert. Hierbei werden zweckmäßig unter kontinuierlicher Zufuhr des 2-Pentensäureesters das Sumpfprodukt zum Sieden erhitzt und der entstandene 3-Pentensäureester fortlaufend vorzugsweise in dem Maße, wie er entsteht, abdestilliert.

Besonders bewährt hat es sich, wenn man cyclische tertiäre Amine verwendet, die einen Siedepunkt oberhalb des jeweils entstehenden 3-Pentensäureesters haben, so daß bei fortwährender Zufuhr von 2-Pentensäureester im Reaktionsgemisch das cyclische tertiäre Amin erhalten bleibt und lediglich 3-Pentensäureester aus dem Reaktionsgemisch entfernt werden. Falls in dem so gewonnenen 3-Pentensäureester restliche Mengen auch 2-Pentensäureester enthalten sind, können letztere in einer zweiten Destillation abgetrennt werden und vorzugsweise in die Isomerisierungsstufe zurückgeführt werden.

Das erfindungsgemäße Verfahren besitzt gegenüber den bis dahin bekannten Verfahren den Vorteil, daß die Umwandlung der 2-Pentensäureester in 3-Pentensäureester schnell, unter milden Bedingungen und mit hoher Selektivität erfolgt. Weitere Vorteile sind die Möglickeit zur Rückgewinnung der Stickstoffbasen und die leichte Aufarbeitung der Reaktionsgemische. Bemerkenswert ist ferner, daß gleichzeitig vorhandene 4-Pentensäureester praktisch nicht verändert werden. Dies ist von Bedeutung, da sich z.B. 2-cis- und 4-Pentensäuremethylester wegen ihres praktisch gleichen Siedepunktes durch Destillation nicht voneinander trennen lassen.

Die nach dem Verfahren der Erfindung erhältlichen 3-Pentensäureester sind wichtige Zwischenprodukte für die Herstellung von Adipinsäure durch Hydroesterifizierung.

Das Verfahren nach der Erfindung sei an folgenden Beispielen veranschaulicht.

Beispiel 1

In einen 1-l-Destillationskolben mit aufgesetzter Füllkörperkolonne (2 m Höhe, Durchmesser 36 mm, 2,1 l Drahtwendeln) und Rücklaufteiler wurde ein Gemisch aus 684 g 2-trans-Pentensäuremethylester (Siedepunkt 137°C/1013 mbar) und 73 g 4-N,N-Dimethylaminopyridin zum Sieden erhitzt. Am Kopf der Kolonne (Rücklaufverhältnis 6:1) wurde ein Pentenestergemisch vom Siedepunkt 127-136°C/1013 mbar abgenommen. In den Sumpf der Kolonne wurden entsprechende Mengen 2-trans-Pentensäuremethylester zudosiert. Innerhalb von 30 Stunden wurden am Kopf der Kolonne 3,936 g Pentenestergemisch abgenommen, das zu 8 % aus 2-cis-Pentensäuremethylester, zu 14 % aus 2-trans-Pentensäuremethylester und zu 78 % aus 3-cis + 3-trans-Pentensäuremethylester bestand.

Beispiel 2

In der im Beispiel 1 beschriebenen Destillationsapparatur wurde ein Gemisch aus 661 g 2-trans-Pentensäuremethylester, 68 g 2-cis-Pentensäuremethylester und 73 g 4-N,N-Dimethylaminopyridin zum Sieden erhitzt. Am Kopf der Kolonne (Rücklaufverhältnis 6:1) wurde ein Pentenestergemisch vom Siedepunkt 125-134°C/1013 mbar abgenommen. In den Sumpf der Kolonne wurden entsprechende Mengen eines Pentenestergemisches zudosiert, das zu 90 % aus 2-trans- und zu 10 % aus 2-cis-Pentensäuremethylester bestand. Innerhalb von 6,5 Stunden wurden am Kopf der Kolonne 704 g Pentenestergemisch abgenommen, das zu 12 % aus 2-cis-, zu 12 % aus 2-trans- und zu 76 % aus 3-cis + 3-trans-Pentensäuremethylester bestand.

Beispiel 3

17,1 g eines Pentenestergemisches, das aus 2-cis- (20 %), 2-trans- (40 %), 3-cis + trans- (20 %), 4-Pentensäuremethylester (20 %) und 0,95 g 1,5-Diazabicyclo[4,3,0]non-5-en bestand, wurde auf 130°C erhitzt. Aus dem Reaktionsgemisch wurde durch Kugelrohrdestillation (60 bis 100°C /20 mbar) das Gemisch der Pentenester vom Katalysator abgetrennt. Die gaschromatographische Analyse ergab, daß das Destillat (16,5 g) zu 2,8 % aus 2-cis-, zu 48,7 % aus 2-trans-, zu 27,9 % aus 3-cis + trans- und zu 19,9 % aus 4-Pentensäuremethylester bestand.

Beispiel 4

Ein Gemisch aus 15 g 2-cis-Pentensäuremethylester und 0,2 g 1,8-Diazabicyclo[5,4,0]undec-7-en wurde auf 130°C erhitzt. Nach 4 Stunden wurde durch Kugelrohrdestillation das gebildete Pentenestergemisch vom Katalysator abgetrennt. Hierbei wurden 14,8 g Destillat und 0,3 g Rückstand erhalten. Das Destillat bestand zu 7 % aus 2-cis-, zu 53,2 % aus 2-trans- und zu 39,6 % aus 3-cis + trans-Pentensäuremethylester.

Beispiel 5

Ein Gemisch aus 20 g 2-cis-Pentensäuremethylester und 2,1 g N,N-4-Dimethylaminopyridin wurde in einem Glasautoklaven eine Stunde auf 180°C erhitzt. Die gaschromatographische Analyse ergab nach dieser Zeit 27,5 % 2-cis-Pentensäuremethylester, 33,7 % 2-trans-Pentensäuremethylester und 38,3 % 3-cis + 3-trans-Pentensäuremethylester. Aus dem Gemisch wurde dann fortlaufend 3-Pentensäuremethylester abdestilliert.

Beispiel 6

Ein Gemisch aus 20 g 2-cis-Pentensäuremethylester und 2,2 g 1,5-Diazabicyclo[4,3,0]non-5-en (DB N) wurde 0,5 Stunden lang unter Rühren auf 130°C erhitzt. Die gaschromatographische Analyse nach dieser Reaktionszeit ergab, daß das Reaktionsgemisch zu 3,2 % aus 2-cis-Pentensäuremethylester, zu 61,3 % aus 2-trans-Pentensäuremethylester und zu 34,2 % aus cis- und trans-3-Pentensäuremethylester bestand. Aus dem Reaktionsgemisch wurde dann fortlaufend 3-Pentensäuremethylester abdestilliert.

Wurde dieser Versuch unter gleichen Bedingungen mit 2-trans-Pentensäuremethylester wiederholt, so setzte sich das Reaktionsgemisch nach 0,5 Stunden Reaktionszeit folgendermaßen zusammen: 3,1 % 2-cis-Pentensäuremethylester, 61,1 % 2-trans-Pentensäuremethylester, 35,7 % cis- und trans-3-Pentensäuremethylester.

Beispiel 7

Wurde Beispiel 6 mit 2,7 % 1,8-Diazabicyclo[5,4,0]undecen-7 (DBU) anstelle von DBN wiederholt, so hatte das Reaktionsgemisch nach 0,5 Stunden bei 130 ° C die folgende Zusammensetzung: 3,5 % 2-cis-Pentensäuremethylester, 63,9 % 2-trans-Pentensäuremethylester und 32,6 % 3-cis + trans-Pentensäuremethylester.

Beispiele 8 bis 12

Die Beispiele 8 bis 12 wurden in gleicher Weise wie Beispiel 5 durchgeführt. Angaben zur Art des eingesetzten Pentenesters, der N-Base, der Reaktionszeit und der Zusammensetzung des Reaktionsaustrages sind in Tab. 1 zusammengefaßt.

| Nr. | eingesetzter PSE1) | verwendete Base | Molverhältnis PSE : Base | Temp. [°C] | Reaktionszeit [h] | PGC (Fl.-%) 2-cis-PSE | 2-tr.-PSE | 3-PSE |
|---|---|---|---|---|---|---|---|---|
| 8 | 2-cis- | | | | | 52 | 6 | 40 |
| 9 | 2-trans- | DABCO2) | | 135 | 4 | 2 | 72 | 22 |
| 10 | 2-cis- | | 1 : 0,1 | 130 | | 79 | 6 | 15 |
| 11 | 2-trans- | DMAP3) | | 130 | 3 | 1 | 86 | 13 |
| 12 | | | | 180 | | 3 | 52 | 37 |

1) PSE = Pentensäuremethylester

2) DABCO = 1,4-Diazabicyclo[2,2,2]octan

3) DMAP = 4-N,N-Dimethylaminopyridin

Beispiel 13

Ein Gemisch aus 22,8 g 2-trans-Pentensäuremethylester und 1,7 g N-Methylimidazol wurde in einem Glasautoklaven eine Stunde auf 180 ° C erhitzt. Die gaschromatographische Analyse ergab nach dieser Zeit 2,0 % 2-cis-Pentensäuremethylester,61,5 % 2-trans-Pentensäuremethylester und 36,3 % 3-cis + 3-trans-Pentensäuremethylester. Aus dem Reaktionsgemisch wurde dann fortlaufend 3-Pentensäuremethylester abdestilliert.

Beispiel 14

Ein Gemisch aus 22,8 g 2-trans-Pentensäuremethylester und 2,0 g N-Methylpiperidin wurde in einem Glasautoklaven 4 Stunden auf 180 ° C erhitzt. Die gaschromatographische Analyse ergab nach dieser Zeit 1,2 % 2-cis-, 72 % 2-trans- und 26,7 % 3-cis + trans-Pentensäuremethylester. Aus dem Reaktionsgemisch wurde dann fortlaufend 3-Pentensäuremethylester abdestilliert.

Vergleichsbeispiel 1

Ein Gemisch aus 11,4 g 2-cis-Pentensäuremethylester und 0,79 g Pyridin wurde in einem Glasautoklaven 4 Stunden auf 180 ° C erhitzt. Die gaschromatographische Analyse ergab nach dieser Zeit 97 % 2-cis-, 0,4 % 2-trans- und nur 2,5 % 3-cis + trans-Pentensäuremethylester.

Vergleichsbeispiel 2

Wie im Beispiel 1 beschrieben, wurde in einen 1 l-Destillationskolben mit aufgesetzter Füllkörperkolonne (2 m Höhe, Durchmesser 36 mm, 2,1 l Drahtwendeln) und Rücklaufteiler ein Gemisch aus 600 g Crotonsäuremethylester (2-trans-Buten-säuremethylester, Siedepunkt 118 bis 120 ° C/1013 mbar) und 91 g 1,8-Diazabicyclo[5,4,0]undecen-7 (DBU) zum Sieden erhitzt. Am Kopf der Kolonne (Rücklaufverhältnis 10 : 1) wurde ein Butenestergemisch vom Siedepunkt 108 bis 112 ° C/1013 mbar abgenommen. In den Sumpf der Kolonne wurden entsprechende Mengen Crotonsäuremethylester zudosiert. Innerhalb von 37 Stunden

wurden am Kopf der Kolonne 615 g Butenestergemisch abgenommen, das zu 61 % aus i-Crotonsäuremethylester(2-cis-Butencarbonsäuremethylester), zu 30 % aus Crotonsäuremethylester und nur zu 9 % aus Vinylessigsäuremethylester bestand.

## Patentansprüche

1. Verfahren zur Herstellung von 3-Pentensäureestern durch Isomerisierung von 2-Pentensäureestern zu 3-Pentensäureestern, dadurch gekennzeichnet, daß man 2-Pentensäureester mit 0,01 bis 0,5 Mol cyclischen tertiären Aminen mit einem $p_{Ka}$-Wert >6 je Mol 2-Pentensäureester bei einer Temperatur von 50 bis 250 ° C behandelt und 3-Pentensäureester fortlaufend abdestilliert.

2. Verfahren nach Anspruch 1, dadurch gekennzeichnet, daß man die Isomerisierung bei einer Temperatur von 100 bis 160 ° C durchführt.

3. Verfahren nach den Ansprüchen 1 und 2, dadurch gekennzeichnet, daß man je Mol 2-Pentensäureester 0,02 bis 0,2 Mol cyclische tertiäre Amine anwendet.

4. Verfahren nach den Ansprüchen 1 bis 3, dadurch gekennzeichnet, daß die verwendeten cyclischen tertiären Amine einen höheren Siedepunkt als die jeweils hergestellten 3-Pentensäureester haben.

5. Verfahren nach den Ansprüchen 1 bis 4, dadurch gekennzeichnet, daß man 5- bis 7-gliedrige tertiäre cyclische Amine, die 1 oder 2 Stickstoffatome oder ein Stickstoffatom und ein Sauerstoffatom im Ring enthalten und die Stickstoffatome jeweils eine Alkylgruppe mit 1 bis 4 Kohlenstoffatomen als Substituenten haben, verwendet.

6. Verfahren nach den Ansprüchen 1 bis 4, dadurch gekennzeichnet, daß man Aminopyridine der Formel I

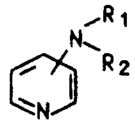

I,

in der $R^1$ und $R^2$ jeweils gleich oder verschieden sind und einen Alkylrest mit 1 bis 4 Kohlenstoffatomen bezeichnen und $R^1$ und $R^2$ gemeinsam mit dem Stickstoffatom, das sie substituieren, einen 5- bis 7-gliedrigen Ring bilden können, der zusätzlich noch ein Sauerstoff-oder Stickstoffatom enthalten kann, verwendet.

7. Verfahren nach den Ansprüchen 1 bis 4, dadurch gekennzeichnet, daß man Imidazole der Formel II

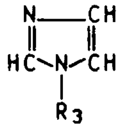

II,

in der $R^3$ einen Alkylrest mit 1 bis 4 Kohlenstoffatomen bezeichnet, verwendet.

8. Verfahren nach den Ansprüchen 1 bis 4, dadurch gekennzeichnet, daß man bicyclische Amidine verwendet.

## Claims

1. A process for the preparation of a 3-pentenoate by isomerization of a 2-pentenoate to a 3-pentenoate, wherein the 2-pentenoate is treated with from 0.01 to 0.5 mole of a cyclic tertiary amine having a $pK_a$ of > 6 per mole of 2-pentenoate at from 50 to 250 ° C and 3-pentenoate is distilled off continously.

**2.** A process as claimed in claim 1, wherein the isomerization is carried out at from 100 to 160 ° C.

**3.** A process as claimed in claims 1 and 2, wherein from 0.02 to 0.2 mole of cyclic tertiary amine is used per mole of 2-pentenoate.

**4.** A process as claimed in claim 1 or 2 or 3, wherein the cyclic tertiary amine used has a boiling point higher than that of the particular 3-pentenoate prepared.

**5.** A process as claimed in claim 1 or 2 or 3 or 4, wherein a 5-membered to 7-membered tertiary cyclic amine which contains 1 or 2 nitrogen atoms or a nitrogen atom and an oxygen atom in the ring, the nitrogen atoms each being substituted by alkyl of 1 to 4 carbon atoms, is used.

**6.** A process as claimed in claim 1 or 2 or 3 or 4, wherein an aminopyridine of the formula I

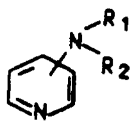

I

where $R_1$ and $R_2$ are identical or different and are each alkyl of 1 to 4 carbon atoms or $R_1$ and $R_2$, together with the nitrogen atom on which they are substituents, may form a 5-membered to 7-membered ring which may additionally contain an oxygen or nitrogen atom, is used.

**7.** A process as claimed in claim 1 or 2 or 3 or 4, wherein an imidazole of the formula II

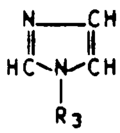

II

where $R_3$ is alkyl of 1 to 4 carbon atoms, is used.

**8.** A process as claimed in claim 1 or 2 or 3 or 4, wherein a bicyclic amidine is used.

**Revendications**

**1.** Procédé de préparation d'esters d'acide 3-penténoïque par isomérisation d'esters d'acide 2-penténoïque en esters d'acide 3-penténoïque, caractérisé en ce qu'on traite des esters d'acide 2-penténoïque par 0,01 à 0,5 mole d'amines tertiaires cycliques ayant une valeur $pK_a>6$ par mole d'ester d'acide 2-penténoïque, à une température de 50 à 250 ° C, et on distille en continu l'ester d'acide 3-penténoïque.

**2.** Procédé selon la revendication 1, caractérisé en ce qu'on conduit l'isomérisation à une température de 100 à 160 ° C.

**3.** Procédé selon la revendication 1 ou 2, caractérisé en ce qu'on utilise, par mole d'ester d'acide 2-penténoïque, de 0,02 à 0,2 mole d'amine tertiaire cyclique.

**4.** Procédé selon l'une quelconque des revendications 1 à 3, caractérisé en ce que les amines tertiaires cycliques utilisées ont un point d'ébullition plus élevé que celui des esters d'acide 3-penténoïques préparés en particulier.

**5.** Procédé selon l'une quelconque des revendications 1 à 4, caractérise en ce qu'on utilise des amines tertiaires cycliques penta- à heptagonales qui contiennent dans le noyau 1 ou 2 atomes d'azote ou un atome d'azote et un atome d'oxygène et présentent, comme substituants sur chacun des atomes d'azote, un groupement alkyle à 1-4 atomes de carbone.

**6.** Procédé selon l'une quelconque des revendications 1 à 4, caractérisé en ce qu'on utilise des aminopyridines de formule I

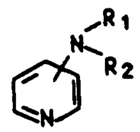

I,

dans laquelle $R^1$ et $R^2$ sont identiques ou différents et désignent chacun un reste alkyle à 1-4 atomes de carbone, $R^1$ et $R^2$ pouvant former ensemble, avec l'atome d'azote qu'ils substituent, un noyau penta- à heptagonal qui peut encore contenir en plus un atome d'oxygène ou d'azote.

**7.** Procédé selon l'une quelconque des revendications 1 à 4, caractérisé en ce qu'on utilise des imidazoles de formule II

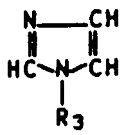

II,

dans laquelle $R^3$ désigne un reste alkyle à 1-4 atomes de carbone.

**8.** Procédé selon l'une quelconque des revendications 1 à 4, caractérisé en ce qu'on utilise des amidines bicycliques.